# EUROPEAN PATENT SPECIFICATION

(11) **EP 3 705 022 B1**
(45) Date of publication and mention of the grant of the patent: **03.04.2024**
(21) Application number: 20159675.6
(22) Date of filing: 27.02.2020
(51) Int. Cl.: A61B 1/00, A61B 1/05

(54) **THERMAL ENDOSCOPE WITH THREE ALIGNED OPTICAL WINDOWS**
THERMISCHES ENDOSKOP MIT DREI IN EINER LINIE POSITIONIERTEN OPTISCHEN FENSTERN
ENDOSCOPE THERMIQUE AVEC TROIS FENÊTRES OPTIQUES ALIGNÉES

(30) Priority: 08.03.2019 IT 201900003347
(43) Date of publication of application: 09.09.2020
(73) Proprietor: Daffara, Claudia, 30100 Venezia (IT); Muradore, Riccardo, 37136 Verona (IT); Siracusano, Salvatore, 34124 Trieste (IT)
(72) Inventor: Daffara, Claudia, 30100 Venezia (IT); Muradore, Riccardo, 37136 Verona (IT); Siracusano, Salvatore, 34124 Trieste (IT); Marchioro, Giacomo, 36034 Vicenza (IT); Scutelnic, Dumitru, 37136 Verona (IT); Fiorini, Paolo, 37131 Verona (IT)
(74) Representative: Gallo, Luca

(56) References cited:
- US-A1- 2011 077 465
- US-A1- 2015 366 461
- US-A1- 2016 006 951
- US-B1- 6 652 452

## Description

The scope of the present invention relates to the sector of devices for the production of infrared images, sensitive to electromagnetic radiation with a wavelength between 8 and 14 microns (LWIR), and their application in the surgical field, in particularly in the field of laparoscopic and robotic surgery.

Surgical operations performed using laparoscopic technique, or laparo-assisted robotics, are now a common practice, since they are advantageous compared to traditional surgical therapy: some of these advantages are quite evident, such as the reduction of the risk of infections, reduced intraoperative blood losses and , in particular, the reduction of postoperative recovery times. For all these reasons laparoscopic and laparo-assisted robotic techniques are used in an increasing number of types of interventions.

They do not foresee the execution of a laparotomy but rather the execution of passageways (doors) with a diameter of the order of one centimeter, between the skin surface and the abdominal cavity, which allow the introduction into the peritoneal cavity, or in the extraperitoneal space, of a duct, called also "trocar", or more ducts inside which the introduction of a specific instrument is made possible, including the endoscope, which allows surgery to be performed. This is made possible thanks to the insufflation of carbon dioxide (CO2) inside the peritoneal cavity or the extraperitoneal space thus allowing the creation of an operating space, necessary for the execution of the operating maneuvers, which are conducted using the instruments inserted through the doors described above.

The interventions in laparoscopy, or in laparo-assisted robotic surgery, provide for the mono and bipolar electrification of the instruments for carrying out the dissection and coagulation of the tissues, which takes place by the release of thermal energy through the use of alternating currents at high frequency. The use of these electrified surgical instruments, and in particular the electrosurgical unit, causes hyperthermia of the areas adjacent to the work area with consequent possible damage to the surrounding nerve structures with possible irreversible functional damage to the related anatomical structures. For this reason, the possibility of monitoring the distribution and diffusion of heat during the use of the electrosurgical unit, or any other instrument that leads to an increase in the temperature of the immediately surrounding tissues, is desirable.

At present, urological interventions, with particular reference to radical prostatectomy, do not provide for monitoring of thermal energy during mono or bipolar coagulation, even if the literature proposes a lot of studies that analyze the risks associated with the use of electrosurgical units in laparoscopy and in laparo-assisted robotic surgery in this surgical context. Among these, reference is made to two studies to attest the great relevance of the problems solved by the present invention.
✔ *"*Complications of electrosurgery in laparoscopy" - Hui-Yu Huang et al. [Gynecology and Minimally Invasive Therapy 3 (2014): 39-42*.*
✔ *"*Principles and Safety Measures of Electrosurgery in Laparoscopy" - Ibrahim Alkatout et al. [ Journal of the Society of Laparoendoscopic Surgeons (2012) 16: 130-139*]*

The present invention, in fact, allows the creation of a thermal endoscope capable of monitoring the temperature of the tissues that are located strictly adjacent to the surgical area concerned. This thermal endoscope, used in combination with the classic optic in the visible, would allow the surgeon to use electrocoagulation near the nerve structures in safe conditions avoiding the irreversible lesion of the latter.

By means to a thermal endoscope such as the one reported in the present invention, for example, it would be possible to carry out nerve-sparing robotic radical surgery on the prostate with the use of targeted bipolar electrocoagulation during dissection of the gland itself. The advantage in these cases would result in a preservation of the nerve structures adjacent to the prostate, reducing the risk of compromising erectile function and patient continence. To date, these risks have a significant impact but are considered inevitable.

A further advantageous use case of the endoscope according to the invention could be the detection of the temperature of the renal parenchyma during robotic or laparo-assisted partial nephrectomy, during the clamping of the renal peduncle, and after the de-clamping of this last, for the determination of the temperature reached at the level of the parenchyma adjacent to the area subjected to enucleoresection, and therefore, indirectly, for obtaining information on the recovery time of the vascularization of the kidney segment taken into consideration.

In the practice of laparoscopic and robotic surgery, however, despite the obvious usefulness, no thermal imaging camera in the *Longwave Infrared* (LWIR) is used, since such an instrument is not currently available.

Indeed, some thermal endoscopes are commercially available (see Thermal Video Endoscope, YTE-T series by Chinese company Yateks) but these cannot be used in the context of laparoscopic and robotic surgery due to their size, sterilization and environmental conditions (remember that in laparoscopic and robotic abdominal surgery, the peritoneal cavity is insufflated with CO2).

In US 2018055335 (A1) [Endoscope Device for Detecting Disease based on Thermal Images - Chung el al. (KR)] an endoscopic device that uses thermal imaging to detect diseases is presented. However, these devices are designed to heat the tissue and measure its cooling, while what is needed to extend the applicability of laparoscopic techniques, as will be further clarified below, it is a passive instrument, as compact as possible, and which allows the acquisition of thermal images with a remarkable definition, capable of measuring temperature increases affecting very small areas of a tissue in a wider field of vision.

Above all, an instrument that can be used in an operative context is needed, while the device described in US 2018055335 (A1) is exclusively aimed at carrying out a specific analysis: both the heating and the detection of the radiation emitted concern only the portion of the examined tissues. Consequently, there are no problems regarding the amplitude of vision (in turn linked to the size and positioning of the sensors) and, in general, there are no problems related to the effectiveness of use of the instrument in an operative context.

In the past, the known art has also proposed the use of suitable instrumentation theoretically suitable for monitoring the temperature of the internal tissues affected by surgical interventions. In this regard, the following are mentioned:
✔ US 6,652,452 B1 "Infrared endoscope with sensor array at the distal tip" - G.J.Seiert et al. (Nov. 25, 2003*);*
✔ US 2017/0065287 A1 "Infrared endoscopic probe" - Silva et al. (Mar.9, 2017*).*

However, surgical practice has not incorporated the use of these tools. It is presumable that having to resort to a double endoscope, the optical and the thermal one, could pose problems, or at least a greater difficulty, from an operational point of view.

Ultimately, it can be concluded that, in surgery, accurate knowledge of the temperature of the tissues is a very important fact that would allow the surgeon to avoid a significant number of complications. However, the prior art has not yet succeeded in proposing a solution accepted by the medical community to meet this need. Therefore, if a solution becomes available, this would certainly be of great benefit, but its development obviously requires the exercise of inventive activity.

As a matter of fact, it should be remembered that as long as the surgical operations are to be supervised by a surgeon, who operates more or less directly the laparoscopic or robotic instruments on the basis of information that must be deciphered in real time, based on what he sees, all the equipment must, first of all, be easy to use and not increase the cognitive load. In particular, the images produced by an endoscope must be clear, synthetic and of immediate intelligibility. Just having to look at two distinct images, the one in the visible to supervise the operating maneuvers, and the thermal one to monitor the temperature, although it is certainly an operation that can be managed by a surgeon, however, constitutes a greater commitment that requires specific concentration.

Regardless of the applications in surgery, thermal image detection technologies are known and based on the acquisition of infrared radiation emitted by the bodies. However, it is not immediate to make a thermal endoscope simply by integrating an infrared radiation detector into a probe suitable for insertion into the patient's body for surgical purposes.

In fact, significant technical problems arise, which therefore require the development of a new instrument, specifically designed for application in surgery.

A first technical problem concerns the need to create a miniaturized device but, at the same time, capable of providing thermal images with a definition suitable for the application. The need for miniaturization is inherent in the nature of endoscopic techniques, the raison d'etre of which is precisely to minimize the invasiveness of surgical interventions. However, small thermal radiation sensors imply a limited number of pixels due to the available space. Just in theory, as a limit case, the sensor could also consist of a single element sensitive to radiation, thus obtaining an extremely small sensor. But such a device would be able to detect a single radiation datum, coming from the zone towards which it is pointed, and measuring the average temperature of this zone, which can also be very small, thus obtaining substantially punctual temperature measurements. This is evidently not acceptable as it would overly engage the surgeon who should maneuver the thermal probe by directing it to the single points where the temperature is important to be evaluated; moreover, not having a thermal image capable of offering a sufficiently broad overview, it would be too difficult, if not impossible, in practice, to perform the temperature measurement exactly at the points of interest. In fact, to monitor the diffusion of heat in order to determine the eventual damage in the surrounding tissues, a map of an area is needed, more precisely a sequence of thermograms over time, and not just a point measurement. Consequently, the miniaturization constraints are very important as they have an impact on the definition of the thermal image that can be obtained and on the width of the field of vision.

The definition of the thermal image is also absolutely important in order to detect excessive temperatures that concern areas of tissue as small as possible. In fact, a tissue's damage, in general, can also be relevant when heating affects a reduced number of cells. On the other hand, the amplitude of the vision is also important as it is necessary to have a thermal image large enough to interpret exactly what is being observed.

All these technical problems, which until a few years ago could not be managed by reaching acceptable performance compromises, can now be faced with better prospects, using the technology of the so-called "thermal cameras" of the new generation, which are spreading thanks to the wave of several possible consumer applications. For example, miniaturized and low-consumption "thermal cameras" can be integrated on mobile terminals (the interest in this technology, in the consumer sector, stems from the fact that a thermal chamber can "see" the presence of people or animals even in the absence of lighting). Therefore, sensors are already available to create "thermal cameras" of very small size, of the order of a centimeter or less, which integrate matrices of pixels, of the order of the various tens, or even of the hundred, per side (or by axis / diameter in the case of non-rectangular generic sensors).

As mentioned, the technological push is dictated by consumer applications, and therefore the application in a niche area, such as endoscopic surgery instrumentation, requires non-trivial adaptations.

In general, with reference to aspects concerning definition and field of vision, it should always be remembered that the instrument must be used during a surgical operation, in which a surgeon must have the most clear information to interpret and the images, in particular, they must be immediately intelligible, so that the surgeon can focus all his attention on the clinical aspects of his surgery.

Further technical problems arise from the need, obviously in surgery, to have an instrument suitable for operating in a sterile environment and therefore suitable for frequent accurate sterilization treatments.

Again, a further technical problem is then determined by the environment in which the thermal endoscope according to the invention must operate, which is characterized by a significant presence of carbon dioxide. The latter, if on the one hand is substantially transparent to radiation in the visible field (and therefore has no impact on the operation of normal optical endoscopes), on the other hand impacts on infrared radiation by distorting the radiation values that reach the sensor (and which can therefore be measured), and therefore the radiometric calculation of the temperature.

Consequently, the general purpose of the present invention is to indicate the essential characteristics of a thermal endoscope suitable for application in surgery, and in particular in laparoscopic and robotic surgery.

Another more specific object of the present invention is to indicate a thermal endoscope of small dimensions which can be integrated into an elongated-shaped casing, such as the casing of a classic optical endoscope whose width must be of the order of one centimeter, and in any case compatible with the use in a surgical operation performed by laparoscopic or laparo-assisted robotic technique.

Another object of the present invention is to indicate a thermal endoscope in which all the necessary components can be hermetically contained in an accurately sterilizable casing.

Another object of the present invention is to indicate a thermal endoscope in which the temperature measurement can be carried out with acceptable precision even in the presence of an infrared radiation propagation environment characterized by a significant presence of carbon dioxide (CO2).

Another fundamental object of the present invention is to indicate a thermal endoscope in which the definition of detection of the infrared radiation, emitted by a tissue whose temperature has to be measured, allows to estimate the temperature with sufficient spatial detail: that is, the individual measurements must refer to areas of tissue as small as possible, in fact, they must be substantially punctual measurements. In addition, the detection field must be large enough to allow the thermal image to be aligned with the visible image that is used by the surgeon to operate. In fact, what is typically most interesting is to monitor the diffusion of heat in the tissues surrounding the point where the scalpel is operating, to evaluate the thermal damage that can be eventually generated during the operation.

The purposes set for this invention are achieved by means of an endoscope for surgical operations according to the subject-matter of independent claim 1.

The main advantage of the present invention is given by the fact that a thermal endoscope made according to the teachings of the present invention, satisfies the purposes for which it was conceived.

This invention also has further advantages which will be made more evident by the following description which highlights further details, by the attached claims which form an integral part of the description itself and by the illustration of some examples of practical embodiment described, by way of non-limiting example, in the following and in the attached drawings in which:
- Figure 1 shows the end part, intended to be introduced into the patient's body, of a thermal endoscope for surgical operations according to the invention;
- Figure 2 shows a broken-view of the thermal endoscope represented in Figure 1 where some internal components are visible.
- Figure 3 shows an exploded view of the thermal endoscope represented in the previous figures which highlights how the device can be assembled and integrated.

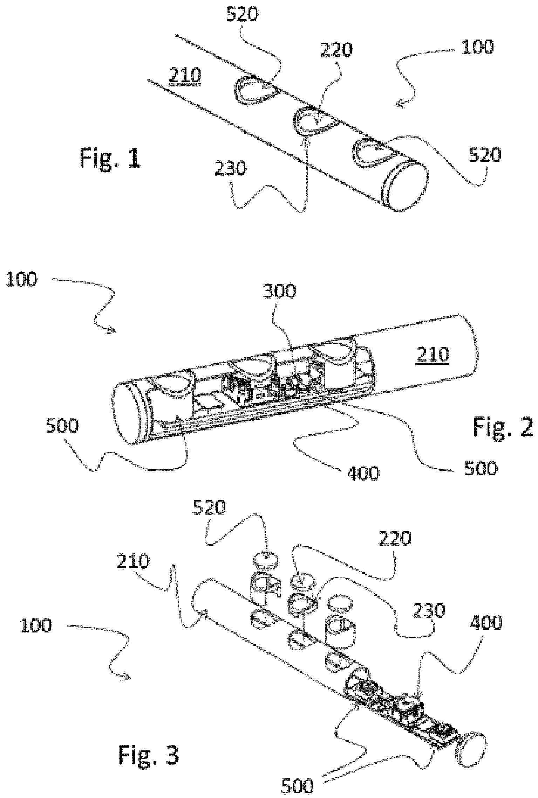

In Figure 1, the number 100 indicates an embodiment of a thermal endoscope according to the invention, of which only the part intended to be inserted into the patient's body during a surgical intervention is seen.

The number 210 indicates the actual casing of the thermal endoscope 100 according to the invention. Typically, it has the shape of a tube with a diameter, as mentioned above, around the centimeter. The casing 210 must be airtight, in order to protect the patient from any type of contamination and, at the same time, protect the internal electronics of the device. Typically, said casing 210 is made of stainless steel or other materials used in the biomedical field for the manufacture of surgical tools.

Said casing 210, in the example of Figure 1, has three holes aligned on one side and positioned near the end of the endoscope 100 intended to be introduced into the patient's body during surgery. These holes are hermetically sealed by three optical windows.

The optical window located in the central hole is indicated with the number 220 and, in the simplest case, it has the function of allowing the infrared radiation, emitted by the tissues towards which it is oriented, to enter the endoscope and invest a particular area inside of the thermal endoscope 100 where, as will be illustrated with the aid of the following figures, a sensor sensitive to radiation in the infrared field is positioned. In more sophisticated cases, said optical window 220, transparent to infrared radiation, can also perform the function of lens, so as to suitably focus the infrared radiation, and improve the detection of the corresponding thermal image (for example, to improve the definition or the field of vision, with the same dimensions).

It should be noted that, although the infrared spectrum is contiguous to the visible spectrum, normal optical glasses do not go well as they shield infrared radiation, therefore said optical window 220 must be made of a suitable material. Germanium is an element with interesting properties thanks to a high level of transparence to radiation between 8 and 14 microns, therefore it is a material that is used with excellent results to make optical articles in the infrared field. Then, a germanium optical window 220 certainly constitutes an adequate choice for a preferred implementation of the invention.

The coupling between said optical window 220, transparent to radiation in the infrared field, and said casing 210 must be particularly cared for ensuring the perfect tightness of the endoscope 100. In fact, neither the bodily substances in contact with the endoscope during the operations must infiltrate inside the endoscope 100, and neither the components inside the endoscope 100 must come into contact, even indirectly, with the patient's body. Hermeticity is also indispensable to ensure the practicability of accurate sterilization treatments to which the external part of the endoscope 100 is subjected. Therefore, normally, the optical window 220 and the actual casing 210 are coupled by means of a suitable gasket, indicated in Figure 1 with the number 230, which ensures the necessary tightness of the object as a whole.

The two optical windows located in the lateral holes are indicated with the number 520, and have the function of allowing the formation of two optical images, in the visible field, in two areas inside the thermal endoscope 100. The positioning of said two optical windows 520, in the visible field, is such that the optical images formed inside the endoscope refer to the same tissues towards which the optical window 220, which allows infrared radiation to pass, is directed.

Even in the case of the optical windows 520, it is possible that these perform the function of lens so as to properly focus the image on the sensitive element. Indeed, in the visible field, the technological maturity is such that optical cameras with extremely miniaturized excellent performance are commonly available (and at low cost), and the optics are often composed of one or more lenses. Therefore, in general, it can be assumed that the optical windows 520 can be simple zones of transparency to the visible, or even real optics made up of suitable lenses. Ultimately, it can be summarized that a prerogative of the thermal endoscope 100, according to the teachings of the present invention, is to provide the surgeon with a visible image of the area in which he is operating (as occurs in normal endoscopes according to the prior art ) which, however, is also enriched with information regarding the temperature.

Therefore, through the two optical windows 520 and the optical window 220 in the infrared field, thermal and optical images referring to the same tissues are simultaneously acquired inside the thermal endoscope 100. It is clear that the shooting points of the different images cannot be physically coincident (therefore the three images, having different points of view, will be slightly different), however, the various windows (or lenses) are carefully positioned in order to acquire images referring to the same tissues. The fact of having two optical images, with different angles, but symmetrical with respect to the angle of recovery of the thermal image, allows to have information that facilitate the reconstruction of an enriched image, which provides, over the image in the visible, also a thermal image superimposed and properly aligned.

Ultimately, the thermal image superimposed on the visible image allows to offer the observer (i.e. the surgeon), through appropriate color rendering, information regarding the temperature of the tissues under observation. For example, the superimposed thermal image can be rendered by attributing specific colors indicative of the temperature to the visible image, or the temperature data can be synthesized to the maximum, highlighting with an appropriate coloring only the tissues that exceed a certain attention temperature , and proposing only the image in the visible (without artificial colors) where the temperature does not exceed attention thresholds. In fact, it is underlined how important it is to know precisely the exact points of the tissues whose temperature reaches levels that can cause contraindications or side effects to be avoided.

It is evident that simplified implementations of the thermal endoscope according to the invention are also possible, in which there is only one optical sensor in the visible. In fact, even if only one image in the visible is available, it is theoretically possible to perform a good alignment between the image in the visible and thermal image, during the "post-processing" phase. However, given that the sensor technology in the visible has now reached, as already mentioned, very high levels of miniaturization, the choice to use a double image in the visible is certainly a preferable implementation. The two cameras in the visible, placed behind the windows / optical lenses 520 allow to provide a stereoscopic vision to the surgeon. The three-dimensional effect of the field of action allows the surgeon to perceive the depth, and to operate in a more natural way to the benefit of the patient.

Again with reference to the form of implementation of Figure 1, it is useful to note that, unlike the endoscopes proposed by the prior art, the image acquisition points (both those in the visible and the thermal ones), represented by the optical windows 520 and 220, are not placed right on the distal end of the endoscope, but on the side of the same, even if, obviously, they are positioned very close to the distal end. This different positioning of the acquisition points, besides being dictated by the fact that, in the case of the present invention, not a single image is acquired (therefore more acquisition points are needed), it also has an additional advantage, in that it allows to use a thermal sensor of slightly larger dimension then the section of the casing 210.

Evaluations conducted with surgeons ensure that the lateral positioning of the endoscope's shooting points is not a problem from an operative point of view, and therefore it is an absolutely feasible choice.

Moreover, the advantage of having all the points of view rigidly united to each other is essential to facilitate the registration and alignment of the images, based on geometric information. If, on the other hand, the thermal image was acquired with a tool that can be operated independently of the optical endoscope, alignment would be much more problematic.

Figure 2 shows a broken-view of the thermal endoscope 100 represented in Figure 1. To make visible some internal parts, a piece of the casing 210 has been eliminated.

Two macro-elements characterizing the invention, which are located inside the thermal endoscope 100, are an electronic board indicated with the number 300 and an infrared radiation sensor indicated with the number 400.

Said electronic board 300 must be designed to perform a plurality of functions, among which the main ones are the following:
- containing the components for the reception, conditioning and adaptation of the signal coming from the sensor 400 and, if the sensor 400 does not include analog-digital conversion electronics inside, said electronic board 300 must sample and quantize the analog signals coming from the sensor itself;
- containing computing means to calibrate the signals detected by the sensor 400 and to build the correct thermal image of the observed area; in particular, a re-mapping of the measured values must be executed, to take into account the effects of infrared propagation in an environment characterized by the presence of carbon dioxide.
- containing the components of the output stages that allow the transfer of the detected thermal images to external devices.

The main functions performed by said electronic board 300 have been illustrated with reference to the management of the signals generated by the thermal sensor 400, however it should be clarified that the thermal endoscope according to the invention also detects normal images in the visible field by means of suitable optical sensors, indicated in Figure 2 with the number 500, and positioned so as to acquire the images focused by the optical windows / lenses 520. Even the signals generated by said optical sensors 500 may require processing which should be carried out immediately downstream of the sensors, therefore by using components to be integrated, in the event, in the electronic board 300. However, since the technology of the optical cameras in the visible is decidedly mature, it is observed that the optical components available on the market are, in many cases, already very integrated, so the signal they produce typically requires less processing.

Finally, the power supply and data transmission cables (or tracks) must be managed and unraveled. All this, with the strict enough constraint to develop the "layout" of the card in the longitudinal direction so that it can be housed in the long and narrow space that is available inside the casing 210 of the thermal endoscope 100. In fact, all the processing means of the analog signal which should be integrated inside the casing 210, as well as the resulting wiring, must be arranged so as to occupy a long and thin space, in which the area of a section orthogonal to the longitudinal direction is in the order of a few square centimeters (indicatively, it can be assumed that this area does not exceed 3 cmq, but it would be better to remain significantly below this limit).

An objective of some interest is that the output signals of the thermal endoscope 100 are digital signals with a well-defined structure and suitable for being directly used by suitable means of visualization; hence the importance of pre-processing the signals at the sensors output.

The infrared radiation sensor 400 is positioned close to the infrared transparent optical window 220, so that the infrared radiation invests it correctly. Infrared radiation sensors can be made with various technologies. Among these, one appears particularly suitable for the application, it is the technology based on the use of micro-bolometers as elementary sensitive elements to constitute the detection of individual "pixels". The arrays of micro-bolometric "pixels" (bolometers are circuits that include a component that varies its resistance according to the radiation that invests them) have, in the state of the art, the advantage of being able to be miniaturized, and not to require a temperature control in order to function properly.

Using a micro-bolometric infrared sensor, it is therefore possible to detect a thermal image consisting of thousands of "pixels", which natively returns an electrical signal, and which requires low power supply.

As anticipated in the first part of this description, dedicated to the prior art, it is reaffirmed that they are already available sensors which, with appropriate adaptations, can be used in the application which is the object of the present invention.

In addition to the calibration necessary to take into account of the particular carbon dioxide-saturated work environment, it is worth highlighting the care that must be taken in sealing and assembling the whole thermal endoscope 100, in which each element must find a place in a small space with a particular shape.

Some assembly measures, important for obtaining a final result that is suitable for achieving the purposes of the invention, are better highlighted with the help of Figure 3.

In Figure 3, the infrared transparent element of the optical window 220 is shown detached from the gasket 230 which, in turn, is detached from the tube-shaped casing 210.

In particular, the gasket 230, in a preferred embodiment, in addition to performing the natural sealing function, also provides the necessary shape adjustments (typically the glasses are flat in shape, also in order not to introduce distorting effects, while the shape of the casing 210 is curved). Obviously, the same observations also apply to the optical lenses 520 in the visible field which, also, must be tightly coupled to the casing 210.

In addition, said gaskets 230 may have a shape which also extends inside the thermal endoscope 100, and also perform other functions for optimizing the assembly, for example by extending up to touching the electronic board 300, or some electronic components connected to it, so as to determine an assembly that is as stable as possible.

When all these elements, which are shown separately in Figure 3, are assembled and grouped, they can in turn be attached to each other, with suitable adhesives, such as, for example, cyanoacrylates for biomedical equipment, or other adhesives suitable for this purpose, so as to form a very compact thermal endoscope 100, hermetically sealed and suitable for being accurately sterilized an indefinite number of times.

In the exploded view of Figure 3, the electronic board 300 is shown outside the casing 210, so as the infrared radiation sensor 400 and the two optical sensors in the visible, indicated with the number 500, are clearly visible.

Of course, other forms of implementation are also possible, what matters is obtaining a usable thermal endoscope, which is compact and elongated and thin, suitable for being inserted into the body of a patient like other instruments used in laparoscopy. At the same time, said thermal endoscope 100 must guarantee the functional performances outlined above, it must be hermetically sealed for repeated accurate sterilization treatments.

Other implementation variants may include the use of a flexible or articulated casing. Obviously, these variants require the availability of suitable materials, and a significant validation and development activity. However, these implementations, if they concern a thermal endoscope with the characteristics set out above, must be considered variants of the thermal endoscope taught in the present invention. Or, if the implementation of these embodiments should require significant exercise of inventive activity, these new embodiments should eventually be considered inventions dependent on the teachings of the present invention.

As can be understood from what has been described above, the invention just described can lend itself to numerous further variants which may offer additional advantages compared to those previously mentioned. And these further variants can be made by the man skilled in the art without however departing from the invention as it results from this description and from the claims attached herein.

Therefore, it may be modified the shape of the thermal endoscope 100 or the shape of some individual constituent elements, such as for example the gasket 230, which in some cases could also be absent as a distinct element, replaced, for example, by suitable glues with sealing function.

Furthermore, each element can be developed in different materials, shapes or sizes; just as the invention itself can be partially implemented and many of the details described can be replaced by technically equivalent elements.

Finally, the described invention is suitable for incorporating and supporting further techniques aimed at further improving the surgical processes for which it is used. In particular, significant developments are expected in electronic components, and in the sector of sensors. Even the sector of automatic image analysis can generate useful developments to improve the performance of the present invention.

All of these expedients and improvements, if they are not included in the present description, may be described in further patent applications, associated with this invention.

## Claims

1. Thermal endoscope (100) for surgical operations suitable for taking an image of an area of tissue inside the body of a patient on which a surgical operation is intended to be performed, which comprises two optical sensors (500) sensitive to radiation in the visible field, wherein
a. said thermal endoscope (100) comprises an end part intended to be inserted in the patient's body during surgery, and three aligned optical windows placed near said end part, and the optical window (220) placed in the center with respect to the other two optical windows (520), which are transparent to the radiation in the visible, is transparent to the infrared radiation;
b. said optical windows (220 and 520) are coupled with the body of said thermal endoscope (100) for surgical operations so as to form a single body properly sealed with respect to the environment in which it must operate;
c. said endoscope (100) for surgical operations also includes an infrared radiation sensor (400) positioned inside;
d. said optical window (220) transparent to the infrared radiation is positioned so as to:
i. let pass through the infrared radiation, which is susceptible to come substantially from the same portion of tissue on which the surgical operation is intended to be performed, and
ii. said infrared radiation is susceptible to hit said infrared radiation sensor (400);
e. said infrared radiation sensor (400) consists of a matrix of elementary sensors that allow the reconstruction of a thermal image;
f. said two optical windows (520) transparent to radiation in the visible are positioned in such a way that said two optical sensors (500) are configured to detect, through said two optical windows (520), two images intended to substantially concern the area of tissue on which the surgical operation is intended to be performed;
wherein said thermal endoscope (100) comprises, inside, electronic processing means and wirings suitable for the transport of signals and suitable for the electrical supply; wherein said thermal endoscope (100) is configured to:
form a stereoscopic image with a three-dimensional effect by means of said two images detected by said two optical sensors (500), and
superimpose said thermal image detected by said infrared radiation sensor (400) on said stereoscopic image so as to provide, by means of a suitable color rendering, information intended to concern the temperature of the tissues whose image is intended to be detected by all these sensors.

2. Thermal endoscope (100) for surgical operations according to claim 1, wherein said optical window (220) transparent to infrared radiation is made of a material whose chemical composition contains the germanium element.

3. Thermal endoscope (100) for surgical operations according to claim 1, wherein said infrared radiation sensor (400) is made with a technology comprising micro-bolometers.

4. Thermal endoscope (100) for surgical operations according to claim 1, wherein said electronic processing means and wiring are arranged so as to occupy a long and thin space, in which the area of a section orthogonal to the longitudinal direction is less than three square centimeters.

## Patentansprüche

1. Thermisches Endoskop (100) für chirurgische Eingriffe, das geeignet ist, ein Bild eines Gewebebereichs im Inneren des Körpers eines Patienten aufzunehmen, auf dem ein chirurgischer Eingriff durchgeführt werden soll, das zwei gegenüber Strahlung im Bereich des sichtbaren Lichts empfindliche optische Sensoren (500) umfasst, wobei
a. das genannte thermische Endoskop (100) ein Ende umfasst, das dazu bestimmt ist, während des chirurgischen Eingriffs in den Körper des Patienten eingeführt zu werden, und drei in der Nähe des genannten Endes in einer Linie positionierte optische Fenster, und das optische Fenster (220) im Verhältnis zu den anderen beiden optischen Fenstern (520), die für Strahlung im Bereich des sichtbaren Lichts durchlässig sind, in der Mitte positioniert und für Infrarotstrahlung durchlässig ist;
b. wobei die genannten optischen Fenster (220 und 520) an den Körper des genannten thermischen Endoskops (100) für chirurgische Eingriffe gekoppelt sind, um einen im Verhältnis zu der Umgebung, in der es operieren soll, entsprechend abgedichteten Körper zu bilden;
c. wobei das genannte Endoskop (100) für chirurgische Eingriffe außerdem einen in seinem Inneren positionierten Infrarotsensor (400) umfasst;
d. wobei das genannte optische Fenster (220), das für Strahlung im Infrarotbereich durchlässig ist, so positioniert ist, dass:
i. es die Strahlung im Infrarotbereich durchlässt, die geeignet ist, im Wesentlichen von demselben Gewebebereich zu kommen, auf dem der chirurgische Eingriff erfolgen soll, und
ii.die genannte Strahlung im Infrarotbereich geeignet ist, auf den genannten Infrarotsensor (400) zu treffen;
e. der genannte Infrarotsensor (400) aus einer Matrix aus elementaren Sensoren besteht, die die Rekonstruktion eines Wärmebildes gestatten;
f. die genannten beiden optischen Fenster (520), die für Strahlung im sichtbaren Bereich durchlässig sind, so positioniert sind, dass die genannten beiden optischen Sensoren (500) darauf ausgelegt sind, über die genannten beiden optischen Fenster (520) zwei Bilder zu erfassen, die dazu bestimmt sind, im Wesentlichen den Gewebebereich zu betreffen, auf dem der chirurgische Eingriff ausgeführt werden soll;
wobei das genannte thermische Endoskop (100) in seinem Inneren elektronische Verarbeitungsmittel und für den Signaltransport geeignete und für die Stromversorgung geeignete Verkabelungen umfasst;
wobei das genannte thermische Endoskop (100) darauf ausgelegt ist:
ein stereoskopisches Bild mit dreidimensionaler Wirkung mittels der von den genannten beiden optischen Sensoren (500) erfassten genannten beiden Bilder zu bilden, und
das von dem genannten Infrarotsensor (400) erfasste genannte Wärmebild über das genannte stereoskopische Bild zu legen, um mittels geeigneter Farbwiedergaben Informationen zu erteilen, die dazu bestimmt sind, die Temperatur der Gewebe zu betreffen, deren Bild dazu bestimmt ist, von allen diesen Sensoren erfasst zu werden.

2. Thermisches Endoskop (100) für chirurgische Eingriffe nach Anspruch 1, wobei das genannte für Infrarotstrahlung durchlässige optische Fenster (220) aus einem Material besteht, dessen chemische Zusammensetzung das Element Germanium enthält.

3. Thermisches Endoskop (100) für chirurgische Eingriffe nach Anspruch 1, bei dem der genannte Infrarotsensor (400) mit einer Mikrobolometer umfassenden Technologie hergestellt wird.

4. Thermisches Endoskop (100) für chirurgische Eingriffe nach Anspruch 1, wobei die genannten elektronischen Verarbeitungsmittel und die Verkabelungen so angeordnet sind, dass sie einen langen und schmalen Raum einnehmen, in dem die Fläche eines zur Längsrichtung orthogonalen Querschnitts weniger als drei Quadratzentimeter beträgt.

## Revendications

1. Endoscope thermique (100) pour opérations chirurgicales apte à capturer une image d'une zone de tissu interne du corps d'un patient au niveau de laquelle une opération chirurgicale est destinée à être effectuée, comprenant deux capteurs optiques (500) sensibles au rayonnement dans le domaine du visible, dans lequel
a. ledit endoscope thermique (100) comprend une extrémité destinée à être insérée à l'intérieur du corps du patient pendant l'intervention chirurgicale, et trois fenêtres optiques alignées positionnées à proximité de ladite extrémité, et la fenêtre optique (220) placée au centre par rapport aux deux autres fenêtres optiques (520), qui sont transparentes au rayonnement dans le domaine du visible, est transparente au rayonnement infrarouge.
b. lesdites fenêtres optiques (220 et 520) sont couplées au corps dudit endoscope thermique (100) pour opérations chirurgicales de manière à former un seul corps convenablement scellé par rapport à l'environnement au sein duquel il doit agir ;
c. ledit endoscope (100) pour opérations chirurgicales comprend également un capteur de rayonnement infrarouge (400) positionné à l'intérieur de celui-ci ;
d. ladite fenêtre optique (220) transparente au rayonnement dans le domaine infrarouge est positionnée de manière à :
i. faire passer le rayonnement dans le domaine infrarouge, qui est susceptible de provenir sensiblement de la même zone de tissu au niveau de laquelle l'opération chirurgicale est destinée à être effectuée, et
ii. ledit rayonnement dans le domaine infrarouge est susceptible de passer à travers ledit capteur de rayonnement infrarouge (400) ;
e. ledit capteur (400) de rayonnement infrarouge est constitué d'une matrice de capteurs élémentaires qui permettent la reconstruction d'une image thermique ;
f. lesdites deux fenêtres optiques (520) transparentes au rayonnement dans le domaine du visible sont positionnées de façon à ce que lesdits deux capteurs optiques (500) soient configurés pour détecter, au moyen desdites deux fenêtres optiques (520), deux images destinées à analyser sensiblement la zone de tissu au niveau de laquelle l'opération chirurgicale est destinée à être effectuée ;
dans lequel ledit endoscope (100) comprend, à l'intérieur de celui-ci, des moyens de traitement électronique et câblages aptes au transport de signaux et aptes à l'alimentation électrique ;
dans lequel ledit endoscope (100) est configuré pour :
former une image stéréoscopique à effet tridimensionnel à l'aide desdites deux images détectées par lesdits deux capteurs optiques (500), et
superposer ladite image thermique détectée par ledit capteur de rayonnement infrarouge (400) à ladite image stéréoscopique de manière à offrir, au moyen de rendus chromatiques appropriés, des informations destinées à analyser la température des tissus dont l'image est destinée à être détectée par tous ces capteurs.

2. Endoscope thermique (100) pour opérations chirurgicales selon la revendication 1, dans lequel ladite fenêtre optique (220) transparente au rayonnement infrarouge est constituée d'un matériau dont la composition chimique contient l'élément dit germanium.

3. Endoscope thermique (100) pour opérations chirurgicales selon la revendication 1, dans lequel ledit capteur (400) de rayonnement infrarouge est réalisé avec une technologie qui comprend des microbolomètres.

4. Endoscope thermique (100) pour opérations chirurgicales selon la revendication 1, dans lequel lesdits moyens de traitement électronique et câblages sont disposés de manière à occuper un espace long et mince, dans lequel l'aire d'une section orthogonale à la direction longitudinale est inférieure à trois centimètres carrés.
